(19) Europäisches Patentamt
European Patent Office
Office européen des brevets

(11) EP 3 537 135 A1

(12) EUROPEAN PATENT APPLICATION

(43) Date of publication:
11.09.2019 Bulletin 2019/37

(51) Int Cl.:
G01N 21/64 (2006.01)     A61M 1/16 (2006.01)

(21) Application number: 18160068.5

(22) Date of filing: 05.03.2018

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Tallinn University of Technology
19086 Tallinn (EE)

(72) Inventors:
• FRIDOLIN, Ivo
  19086 Tallinn (EE)
• KALLE, Sigrid
  19086 Tallinn (EE)
• TANNER, Risto
  19086 Tallinn (EE)

(74) Representative: Koppel, Mart Enn
KOPPEL patendibüroo OÜ
Oja tee 22-2, Tiskre, Harku vald
76916 Harjumaa (EE)

(54) A DEVICE AND METHOD FOR ASSESSMENT OF A CONCENTRATION OF FREE PENTOSIDINE IN A SPENT DIALYSATE

(57) A novel method and a device for assessment of the concentration of advanced glycation end products (AGE), such as pentosidine in particular, in the biological fluids, such as spent dialysate. More specifically, the present invention relates to an optical method utilizing fluorescence and a specific model, including a unique set of optical spectral components at certain wavelengths, to determine, preferable on-line, the concentration of an AGE compound, such as pentosidine.

Fig. 7

**Description**

**Technical field**

This invention relates to a novel method and a device for assessment of the concentration of advanced glycation end products (AGE), such as pentosidine in particular, in the biological fluids, such as spent dialysate. More specifically, the present invention relates to an optical method utilizing fluorescence and a specific model, including a unique set of optical spectral components at certain wavelengths, to determine, preferable on-line, the concentration of an AGE compound, such as pentosidine.

**Background art**

Pentosidine was first described in 1989 by Sell and Monnier and it consists of arginine and lysine residues that are crosslinked by pentose [1]. This compound is considered to be a uremic toxin and is listed in the protein-bound group of the uremic toxins database [2].

Pentosidine, a glycoxidation product of proteins, belongs to advanced glycation end products (AGE) group [3]. AGEs tend to accumulate in uremic patients' plasma as the result of oxidative stress and decreased excretion. AGE accumulation in chronic kidney disease (CKD) patients may lead to chronic complications [4].

More than 95% of plasma pentosidine is protein bound [5] and only 3-4% is free circulating pentosidine in a healthy individual [4] [6]. Concentration of free pentosidine will start to increase in patients with declined renal function [2] [7]. We have found that in spent dialysate 79-84% of pentosidine is in free form [8]. Machowska et al. (2016) found that pentosidine concentrations were higher in HD patients than in PD patients and that in different stages of CKD there are different contributors that increase plasma pentosidine levels [9]. It has been found that higher plasma pentosidine levels are associated with inflammation [5] [9] [10], malnutrition [10], oxidative stress [9] and low GFR [9].

AGEs are potential uremic toxins [11] and therefore following the removal of AGEs during dialysis can be used as an indicator of the therapy. As plasma pentosidine correlates strongly with the levels of precursors of carbonyl compounds, it is perceived as a good marker substance for AGE and oxidative stress [5].

Pentosidine is mainly measured by high-pressure liquid chromatography (HPLC) [1] [10] [7] [5] [12] and for selectivity reverse-phase (RP) C18 column is used [1] [10] [7] [5]. Ni et al. (2009) have also used special porous graphite carbon column [12]. For HPLC measurements mainly mixtures of water and acetonitrile [1] [5] [10] [7] [12] have been used for elution with ion-pair modifiers heptafluorobutyric acid (HFBA) [1] [10] or trifluoroacetic acid (TFA) [12]. Plasma pentosidine can be measured with ELISA or RP-HPLC which requires plasma hydrolysis. Both methods have shown comparable results, but HPLC is more accurate because ELISA methods may not identify pentosidine on the inside of proteins [3]. Moreover, if ELISA is applied after digestion of protein, the antibodies may not recognize the altered epitope [3]. These methods are laborious for use in clinical practices, need blood samples and expensive reagents. In regard to treatment of CKD patients, these methods do not give information about the ongoing HD session.

Real-time optical measurement of spent dialysate is successfully used to monitor uric acid and indoxyl sulfate during HD treatment of CKD patients [13] [14] [15].

EP2585830 discloses a method and device for determining content of the middle and protein bound uremic toxins in a biological fluid using an optical method utilizing fluorescence, preferable fluorescence of the spent dialysate, and a specific model, including a unique set of optical spectral components at certain wavelengths, to determine, preferable on-line, the concentration of the middle and protein bound uremic toxins, such as beta2-microglobulin (B2M), and indoxyl sulfate (IS). The invention discloses AGE's and lists pentasidine as one of the AGE's, but do not disclose any details how the concentration of AGE's and free pentosidine in particular in spent dialysate could be assessed.

What is needed, therefore, is a method for assessing the concentration of free pentosidine in a biofluid such as spent dialysate, preferably, on-line, by fluoresence measurements.

**Summary of invenion**

These and other objectives of the invention are achieved by a device for assessing an concentration of free pentosidine in a biofluid, such as spent dialysate, comprising:

ultraviolet light means for generating excitation light and guiding said light into said biofluid, said light causing a flouresence emission of at least free pentosidine in said biofluid,

ultraviolet sensor means for receiving and sensing said fluoresence light emission, and

processing means for calculating concentration of free pentosidine based on intensity of said fluoresence light emission,

wherein said ultraviolet light means are adapted to irradiate excitation light with wavelenght from 330 to 350nm, preferably 350nm and said ultraviolet sensor means are adapted to sense fluoresence emission light with wavelenght is from 360 to 385nm, preferably 363nm.

In addition to single wavelenght (SW) measurements, the device can be further preferably adapted to carry out multi wavelenght (MW) measurement, wherein said excitation light has wavelengths 350nm, 250nm and 430nm, and said fluoresence emission light has wavelengths 363nm, 321nm and 463nm, respectively.

The device preferably comprises means for receiving flowing spent dialysate, e.g., a fluorometrical flow-cuvette. Such flow-cuvettes are wellknown in the art. Alternatively, the device may comprise in vitro cuvette for receiving a sample of said biofluid.

These and other objectives of the invention are also achieved by a method for assessing a concentration of a free pentosidine in a biofluid, such as spent dialysate, comprising:

introducing ultraviolet light with wavelength from 330 to 350 nm, most preferably, 350nm, into said biofluid, said light causing a flouresence emission of at least free pentosidine in said biofluid,

receiving said fluoresence light emission with wavelength 360 to 385nm, preferably 363nm from said biofluid, and

determining said concentration of said free pentosidine from the intensity of said fluoresence light emission.

Preferably, the method further comprising introducing additionally ultraviolet light with wavelenghts 250nm and 430nm into said biofluid, and receiving additionally fluoresence light with wavelengts 321nm and 463nm, respectively.

**Brief description of drawings**

Fig. 1 is HPLC chromatograms of spent dialysate (patient #22) and of standard pentosidine.

Fig. 2 depicts the fluorescence spectrum of standard pentosidine in 0.050M HFBA in MilliQ water. It can be seen that the fluorescence maximum is at the wavelength range of Ex320-330/Em360-385 nm.

Fig. 3 shows correlation coefficient values between fluorescence of spent dialysate and concentration of free pentosidine. The correlation maximum is at Ex350/Em363 nm.

Fig. 4 shows emission spectrum of standard pentosidine (solid line) and correlation coefficients of SW model (dashed line) at excitation 330nm (A) and 350nm (B).

Fig. 5 shows free pentosidine concentration determined at the laboratory and predicted by the SW (A) and MW (C) model, and Bland-Altman plot of the differences between Lab and SW (B), Lab and MW (D) concentrations.

Fig. 6 is a Bland-Altman plot of the differences between A) RR_lab and RR_SW and B) RR_lab and RR_MW.

Fig. 7 is a device according to one embodiment of the invention.

**Examples of carrying out the invention**

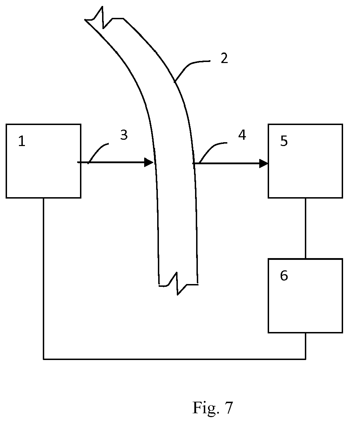

The device according to the invention is in Fig. 7, comprising an ultraviolet light means 1 for generating excitation light 2 and guiding said light into said biofluid in a flow-cuvette 3, said light causing a flouresence emission 4 of at least free pentosidine in said biofluid, an ultraviolet sensor means 5 for receiving and sensing said fluoresence light emission, and processing means 6 for calculating a concentration of a free pentosidine based on intensity of said fluoresence light emission. Said processing means is adapted to execute concentration calculation algorithm, e.g., a hemodialyses (HD) or haemodiafiltration (HDF) concentration or removal calculation algorithm comprising a transforming function calculating the concentration of pentosidne in the biological fluid. Said ultraviolet light means are adapted to generate excitation light with wavelenght from 330 to 350nm, preferably 350nm while said ultraviolet sensor means are adapted to sense fluoresence emission light with wavelenght is from 360 to 385nm, preferably 363nm. Either narrow band light sources, well known in the art, can be used, or wide band light sources with appropriate filters, also well known in the art, can be used. Either narrow band light sensors can be used, or wide band light sensors with appropriate filters can be used, both well known in the art.

Experimental

Forty patients (age 61.3 ±16.3 years) during 40 dialysis sessions were followed. 33 patients received haemodiafiltration (HDF) and 7 hemodialysis (HD) treatment.

Fresenius 5008 or 4008H (Fresenius Medical Care, Germany) machine was used for the dialysis. Dialyzers were FX8, FX100, FX800 and FX1000 and the dialysate and blood flow varied from 204 - 800 mL/min and 170 - 360 mL/min, respectively. Treatment durations varied from 180 - 247 minutes.

*A. Sampling and laboratory analysis*

During each dialysis two samples were collected from the outlet dialysate line - at the start and end of the dialysis session.

High pressure liquid chromatography (HPLC) was used to measure free pentosidine concentrations. The HPLC system consisted of Ultimate 3000 Series instruments from Dionex (Sunnyvale, USA) and of Kinetex core-shell column 2,6 $\mu$m C18 150*4.6 mm with the guard AJ0-4287 4*3 mm, both Phenomenex, USA. Chromatographic data processing was done with Chromeleon 7.1.2.1478 software by Dionex Thermo Scientific (Waltham, USA). For the analysis HPLC grade acetonitrile (AcN, (Honeywell, USA)) and HFBA (Sigma-Aldrich, USA) solvents were used. The solvent flow was 0.8 ml/min and the column temperature was 35.0 ±1.0 °C. All spent dialysate samples were acidified down to pH 4.25 with formic acid before the HPLC analysis. Two-component eluent was used for the chromatographic analysis as mixture of A: 0.050M HFBA in MilliQ water and B: 0.005M HFBA in AcN. The five-step linear gradient elution program was used, as specified in Table 1.

Table 1. HPLC gradient program.

| Step | Time (min) | Buffer A, % | Buffer B, % | Curve type |
|------|-----------|-------------|-------------|------------|
| 0 | 0 | 99 | 1 | 5 |
| 1 | 4 | 99 | 1 | 5 |
| 2 | 34 | 10 | 90 | 8 |
| 3 | 44 | 10 | 90 | 5 |

The chromatograms were recorded with RF 2000 fluorescence detector Dionex (Sunnyvale, California, USA) using excitation 330 and emission 373 nm. Pentosidine with purity >98% (Cayman Chemical (Ann Arbor, Michigan, USA)) was used as a reference for building up calibration curve. The concentration of the calibration solution was estimated on the basis of UV-absorbance using molecular extinction coefficient 4522 AU/[(mol/l) cm] [20].

UV-absorbance spectra of dialysates were recorded with a double-beam spectrophotometer (Shimadzu UV-2401 PC, Japan) over the wavelength range of 190 - 380 nm using an optical cuvette with an optical path length of 4 mm.

Full fluorescence spectra of spent dialysates in the range of excitation/emission 220-490 nm with excitation increment 10 nm were recorded with the spectrofluorophotometer RF-5301 by Shimadzu (Kyoto, Japan). Measurements were done with optical cuvette of 4 mm optical path length and data processing was done with the Panorama Fluorescence 1.2 software by Shimadzu.

*B. Data analysis*

Forward step-wise regression analysis was used to acquire a single wavelength (SW) and a multi-wavelength (MW) model for the assessment of free pentosidine concentration through fluorescence intensity measurements. Independent variables included were fluorescence intensity values at the wavelengths Ex/Em 220-490. It was presumed that independent variables are linearly independent, and that free pentosidine concentration is related to fluorescence intensity. Patient-dependent parameters (such as BMI, age, gender and smoking) were added to the models but it turned out to be statistically insignificant and were not included in the models. The data was divided into calibration set (75% of the data) which was used to develop the algorithm and validation set (25% of the data) which was used to validate it.

Systematic error (*BIAS*) was calculated as follows:

$$BIAS = \frac{\sum_{i=1}^{N}(c_{average}-c_i)}{N} \qquad (1)$$

where c is the concentration of free pentosidine in spent dialysate and N is the number of observations [16].

Standard error (SE) was calculated by the following formula [16]:

$$SE = \sqrt{\frac{\sum_{i=1}^{N}(e_i-BIAS)^2}{N-1}} \qquad (2)$$

where $e_i$ is the difference of lab and model concentration for the i-th measurement.

SpektroModel software [17] (Michelis, Estonia) was used for the analysis of data designed to generate regression models from spent dialysate fluorescence spectra for predicting concentrations of uremic solutes. Excel (version 2016 for Mac and Windows) was used for graph generation.

The reduction ratio (RR) of free pentosidine for a dialysis treatment was calculated from the start ($C_{start}$) and the end ($C_{end}$) concentrations from the dialysis session as:

$$RR(\%) = \frac{C_{start}-C_{end}}{C_{start}} * 100 \qquad (3)$$

The RR was calculated based on laboratory values (RR_lab) as well as from the concentrations estimated by the SW model (RR_SW) and MW model (RR_MW). The student's t-test was applied to compare means for RR_lab, RR_SW, RR_MW and $p < 0.05$ was considered significant. Bland-Altman analysis [18] was used to examine the individual differences in RR_SW and RR_MW compared to RR_lab values.

**Results**

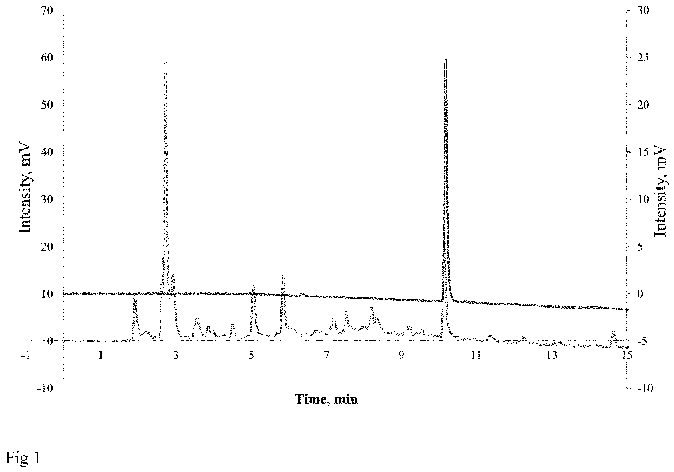

An example of HPLC chromatograms of spent dialysate of patient #22 and of standard pentosidine can be seen in figure 1.

Average free pentosidine concentration in the beginning and in the end of the dialysis sessions are shown in table 2.

Table 2. Average free pentosidine concentrations ($\mu$g/L) of the dialysis sessions

|  |  | Lab | SW | MW |
|---|---|---|---|---|
| Calibration | Beginning | $4.25 \pm 3.11$ | $4.47 \pm 2.60$ | $3.94 \pm 3.24$ |
|  | End | $0.94 \pm 0.69$ | $0.63 \pm 0.30$ | $1.02 \pm 0.69$ |
| Validation | Beginning | $4.50 \pm 4.01$ | $4.82 \pm 2.85$ | $4.58 \pm 3.27$ |
|  | End | $1.07 \pm 0.85$ | $0.69 \pm 0.25$ | $1.21 \pm 0.69$ |

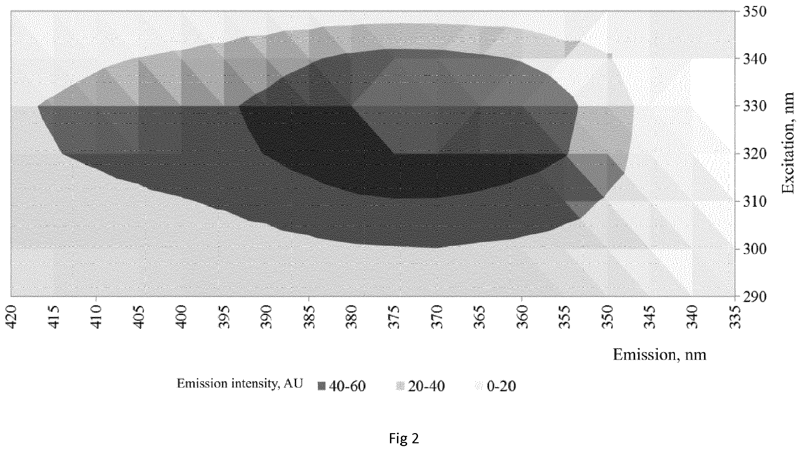

Figure 2 depicts the fluorescence spectrum of standard pentosidine in 0.050M HFBA in MilliQ water. It can be seen that the fluorescence maximum is at the wavelength range of Ex320-330/Em360-385 nm. The fluorescence maximum is at Ex330/Em370 nm.

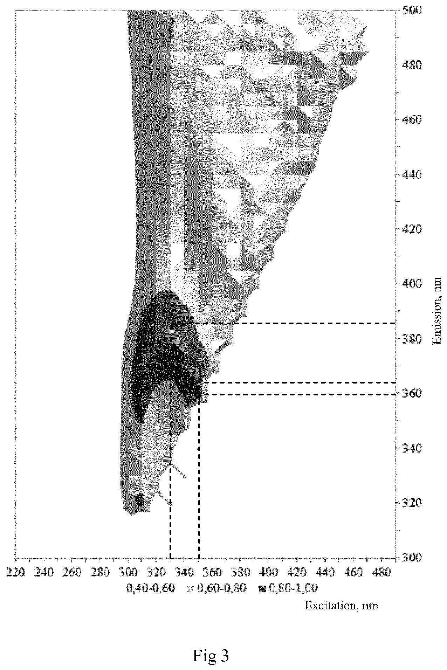

Figure 3 shows the correlation coefficient (R) values between fluorescence of spent dialysate over the wavelength of Ex220-480/Em230-490nm and concentration of free pentosidine (HPLC results). It can be seen that the highest correlation between fluorescence of spent dialysate and free pentosidine concentration is around Ex340-350/Em360-385 nm with the maximum at Ex350/Em363 nm).

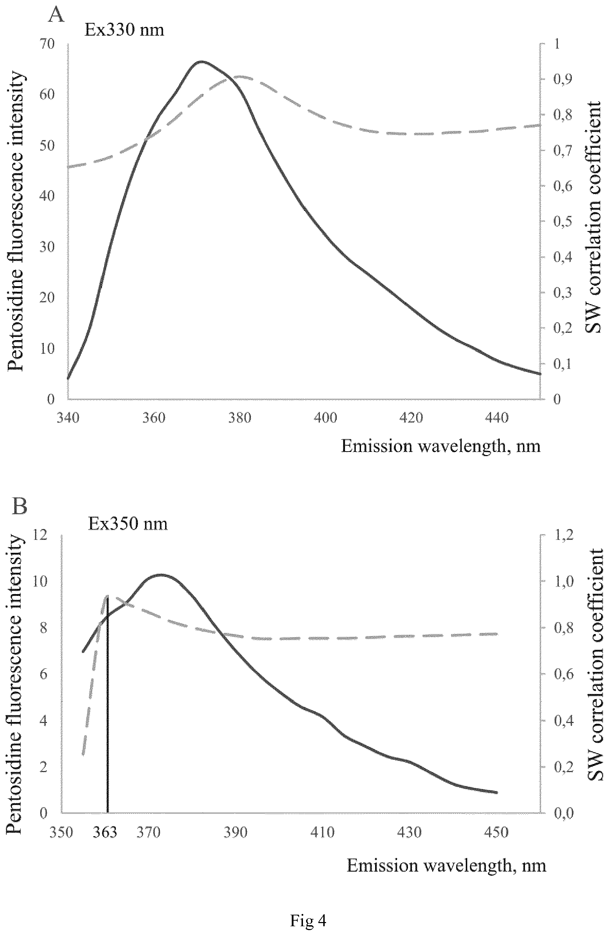

The comparison between fluorescence of standard pentosidine and correlation coefficients at excitation 330 nm and 350 nm is shown in figure 4.

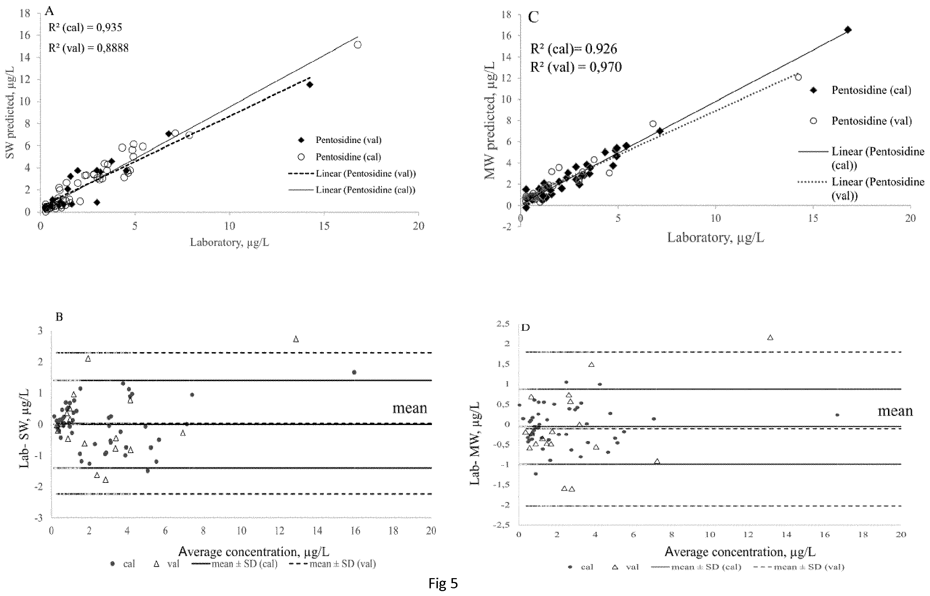

The scatter and Bland-Altman plots of free pentosidine concentration predicted by the SW model at the wavelength of the correlation maximum can be seen on figure 5A and 5B. The SW model gave high correlation coefficient R of 0.966 and 0.943 for cal and val sets, respectively.

The multi-wavelength model (MW) included the fluorescence wavelengths Ex350/Em363 nm, Ex250/Em321 nm and Ex430/Em463 nm with the general formula: pentosidine concentration= a+(b*Ex350/Em363)+(c*Ex250/Em321)+(d*Ex430/Em463), wherein a, b and c are coeficients with value between -1 and 1 and are determined by skilled person from calibration set of data using known methods. Figs 5C and 5D show the scatter and Bland-Altman plots of the concentration of free pentosidine predicted by MW model for the calibration and validation set. The MW model gave even higher correlation coefficients R of 0.985 and 0.962 for cal and val sets, respectively, than SW model.

The correlation coefficients, coefficients of determination, the number of cases, the standard error (SE) and BIAS of the models for the calibration and validation set are presented in Table 3. For the calibration and validation set the highest R and $R^2$ value was obtained with the MW model compared to the SW model. However, BIAS was the lowest for the SW model. BIAS values for the calibration and validation set of SW and MW models were not statistically different (p>0.6). SE was the lowest for MW model for both sets. SE values between the SW and MW models were statistically different (p=0.03) for calibration set but not for validation set ($p$=0.54).

Table 3. The number of cases (N), the standard (SE) and systematic (BIAS) errors of the models, correlation coefficients (R), and coefficients of determination ($R^2$) for the calibration and validation sets.

|  | Set | N | BIAS | SE | R | $R^2$ |
|---|---|---|---|---|---|---|
| SW | Calibration | 53 | 0.000 | 0.702 | 0.966 | 0.934 |
|  | Validation | 18 | 0.031 | 1.135 | 0.943 | 0.889 |
| MW | Calibration | 50 | -0.055 | 0.464 | 0.985 | 0.970 |
|  | Validation | 18 | -0.109 | 0.962 | 0.962 | 0.926 |

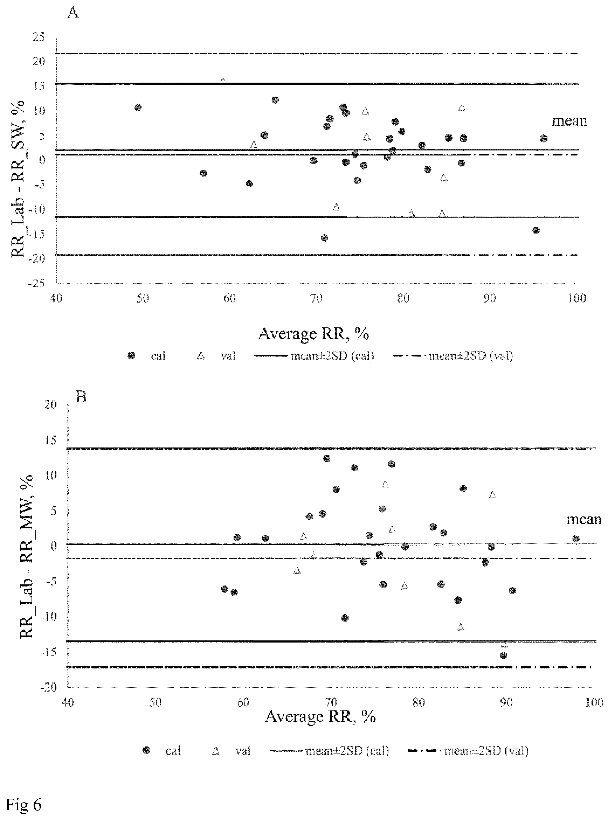

The average removal ratio of free pentosidine for cal and val set can be seen in table 4. Small differences between RR_lab and RR_SW, RR_MW were not statistically different (p>0.05 cal and val set). Differences between RR_lab and RR_SW and between RR_lab and RR_MW can be seen from the Bland-Altman plot on figure 6A and 6B, respectively.

Tabel 4. The average RR removal ratio of free pentosidine pentosidine estimated with SW and MW models: (N=36).

|  | RR_Lab | RR_SW | RR_MW |
|---|---|---|---|
| Calibration | 76.4±10.3% | 74.4±11.7% | 76.2±11.4% |
| Validation | 76.4±8.8% | 75.3±12.8% | 78.1±10.8% |

Fig 6 shows. Bland-Altman plot of the differences between A) RR_lab and RR_SW and B) RR_lab and RR_MW.

The mean difference between RR_lab and RR_SW was -2.0±6.8% for the cal set and -1.1± 10.2% for the val set. The mean difference between RR_lab and RR_MW was -0.17 ± 6.8% for the cal set and 1.8±7.7% for the val set.

Relationship between concentration of free pentosidine and fluorescence intensity of spent dialysate was studied and single- (SW) and multi-wavelength (MW) models were developed for the evaluation of the free pentosidine concentrations in spent dialysate and removal ratio during dialysis.

It is feasible to monitor elimination of free pentosidine during dialysis by using fluorescence of the spent dialysate even when the technique evidently measures several other fluorophores and chromophores in spent dialysate at the same time. The MW algorithm slightly improves estimation of the free pentosidine concentration and RR estimation accuracy in contrast to the SW algorithm. The highest $R^2$ value for calibration and validation set were obtained with the MW model (table 3). Slightly lower $R^2$ values for both sets were obtained with SW model. BIAS values of SW and MW model were not statistically different (p>0.05). However, SE was the lowest for MW model for the calibration and validation set. SE values between the SW and MW models were statistically different (p=0.03) for calibration set. The high SE values maybe due to low sample size.

Recently, a device for measurement of skin autofluorescence (SAF) was developed for assessment of the AGE level of patients [18]. No association between SAF and total plasma pentosidine has been found in mortality studies on CKD patients [9]. Our previous study [19] found relatively high correlation between SAF and fluorescence of spent dialysate. This observation opened an attractive prospect for assessment of free pentosidine removal from CKD patients by measurement of fluorescence properties of dialysates as an easily available alternative to complicated HPLC or ELISA methods. Two different assessment models were comprised for this purpose.

The wavelength of the maximum fluorescence of the standard pentosidine (Fig 2) is in good agreement with corresponding data in literature (Ex330/Em373 [20]). The SW model includes the fluorescence wavelength at the correlation maximum between free pentosidine concentration and fluorescence of spent dialysate (Fig. 3). However, surprisingly, it did not exactly correspond to the fluorescence maximum value of standard pentosidine (Figs 2,3). Evidently, there may appear interferences of other fluorophores which shift the correlation maximum to a different wavelength pair (Fig. 4).

Pentosidine is considered to be a marker for AGE-modified proteins in the body fluids. In this reason, pentosidine is very exceptional among other protein bound uremic toxins and also different from water soluble uremic solutes like uric acid. Pentosidine removal does not reflect status in body fluids in the same way as it does for small water soluble uremic solutes [23]. The most of pentosidine is known to be covalently bound in the structure of AGE-modified tissue proteins and cannot penetrate the dialysis membrane. For this reason, we consider pentosidine as a unique marker for dialysis adequacy monitoring. Since HD fails to lower total plasma concentration of protein-bound pentosidine [4] a valid alternative would be to follow the removal of free pentosidine. Miyata et al. [4] found the reduction ratio of free pentosidine to be more than 75% with HD. This corresponds to results of our study where we found an average RR_lab of $76.4 \pm 10.3\%$ and $76.4 \pm 8.8\%$ for the cal and val set, respectively (table 3). The best RR estimation was achieved with the MW model, where average RR was $76.2 \pm 11.4\%$ and $78.1 \pm 10.8\%$ for the cal and val set, respectively. The SW model slightly underestimated the average RR values.

MW model presented slightly more accurate results than the SW model.

Free pentosidine concentration estimation in spent dialysate and removal assessment from CKD patients during dialysis using measurements of fluorescence of spent dialysate is feasible. The advantages of the method are that it does not need blood sampling, chemicals and is fast.

**Acknowledgement**

The study leading to the invention was supported by the European Union through the European Regional Development Fund and the Estonian Ministry of Education and Research under institutional research funding IUT19-02.

References

[1] D. R. Sell and V. M. Monnier, "Structure Elucidation of a Senescence Cross-Link from Human Extracellular Matrix," no. 36, pp. 21597-21602, 1989.

[2] Vanholder, "Uremic Toxin - Data Base," 09 2009. [Online]. Available: http://www.uremic-toxins.org/Data-Base.html. [Accessed 09 2017].

[3] M. E. Suliman, O. Heimburger, P. Barany, B. Anderstam, R. Pecoits-Filho, E. R. Ayala, A. R. Qureshi, I. Fehrman-Ekholm, L. B and P. Stenvinkel, "Plasma Pentosidine Is Associated with Inflammation and Malnutrition in End-Stage Renal Disease Patients Starting on Dialysis Therapy," vol. 6, pp. 1614-1622, 2003.

[4] T. Miyata, Y. Ueda, A. Yoshida, S. Sugiyama, Y. Iida, M. Jadoul, K. Maeda, K. Kurokawa and C. van Ypersele de Strihou, "Clearance of pentosidine, an advanced glycation end product, by different modalities of renal replacement therapy," Kidney International, vol. 51, no. 3, pp. 880-887, 1997.

[5] T. Miyata, Y. Ueda, T. Shinzato, Y. Iida, S. Tanaka, K. Kurokawa, C. van Ypersele de Strihou and K. Maeda, "Accumulation of albumin linked and free-form pentosidine in the circulation of uremic patients with end-stage renal failure: Renal implications in the pathophysiology of pentosidine," Journal of the American Society of Nephrology, no. 7, p. 1198-1206, 1996.

[6] M. A. Friedlander, Y. C. Wu, A. Elgawish and V. M. Monnier, "Early and advanced glycosylation end products. Kinetics of formation and clearance in peritoneal dialysis," Journal of Clinical Investigation, vol. 97, p. 728-735, 1996.

[7] G. Stein, S. Franke, A. Mahiout, S. Schneider, H. Sperschneider, S. Borst and J. Vienken, "Influence of dialysis modalities on serum AGE levels in end-stage renal disease patients," Nephrology Dialysis Transplantation, vol. 16, no. 5, pp. 999-1008, 2001.

[8] M. Adler, "Pentosidine Analysis Method Implementation in Chromatography Laboratory of TUT Department of Health Technologies," TTU, Tallinn, 2017.

[9] A. Machowska, J. Sun, A. R. Qureshi, N. Isoyama, P. Leurs, B. Anderstam, O. Heimburger, P. Barany, P. Stenvinkel and B. Lindholm, "Plasma Pentosidine and Its Association with Mortality in Patients with Chronic Kidney Disease," PLOS one, 2016.

[10] M. E. Suliman, O. Heimbürger, P. Barany, B. Anderstam, R. Pecoits-Filho, A. E. Rodriguez and e. al., "Plasma pentosidine is associated with inflammation and malnutrition in end-stage renal disease patients starting on dialysis therapy," Journal of the American Society of Nephrology, vol. 6, no. 16, p. 1614-1622, 2003.

[11] A. Heidland, K. Sebekova and R. Schinzel, "Advanced glycation end products and the progressive course of renal disease," American Journal of Kidney Diseases, no. 34, p. S100-S106, 2001.

[12] J. Ni, X. Yuan, J. Gu, X. Yue, X. Gu, R. H. Nagaraj and J. W. Crabb, "Plasma Protein Pentosidine and Carboxymethyllysine, Biomarkers for Age-related Macular Degeneration," Molecular and Cellular Proteomics, vol. 8, no. 8, pp. 1921-1933, 2009.

[13] F. Uhlin, J. Holmar, P. Yngman-Uhlin, A. Fernström and I. Fridolin, "Optical Estimation of Beta 2 Microglobulin During Hemodiafiltration - Does It Work?," Blood Purification, vol. 2, no. 40, pp. 113-119, 2015.

[14] I. Fridolin, M. Magnusson and L. G. Lindberg, "On-Line Monitoring of Solutes in Dialysate Using Absorption of Ultraviolet Radiation: Technique Description," The International journal of Artificial Organs, vol. 8, no. 25, pp. 748-761, 2002.

[15] S. Kalle, R. Tanner, J. Arund, R. Tomson, M. Luman and I. Fridolin, "4-Pyridoxic Acid in the Spent Dialysate: Contribution to Fluorescence and Optical Monitoring," PLOS one, 2016.

[16] K. H. Esbensen, Multivariate data analysis - in practice, Oslo, Norway: CAMO Process AS, 2009.

[17] J. Michelis, Master thesis: Software for regression analysis of biofluids' spectrophotometric data, Tallinn University of Technology, 2016, p. 59.

[18] J. M. Bland and D. G. Altman, Statistical methods for as - sessing agreement between two methods of clinical measurement, Lancet, 1986.

[19] S. Kalle, R. Tanner, I. Fridolin and M. Luman, "Association between skin autofluorescence and fluorescence of spent dialysate," in Nephrology Dialysis Transplantation, 54th ERA-EDTA Congress. 3-6 june 2017

[20] M. E. Suliman, O. Heimbürger, P. Bárány, B. Anderstam, R. Pecoits-Filho, E. Rodriguez Ayala, A. R. Qureshi, I. Fehrman-Ekholm, B. Lindholm and P. Stenvinkel, "Plasma pentosidine is associated with inflammation and malnutrition in end-stage renal disease patients starting on dialysis therapy," Journal of the American Society of Nephrology, vol. 6, no. 14, pp. 1614-1622, 2003.

[21] D. Slowik-Zylka, K. Safranow, V. Dziedziejko, H. Bukowska, K. Ciechanowski and D. Chlubek, "A sensitive and specific HPLC method for the determination of total pentosidine concentration in plasma," Journal of Biochemical and Biophysical Methods, no. 61, pp. 313-329, 2004.

[22] R. Meerwaldt, J. W. Hartog, R. Graaff, R. J. Huisman, T. P. Links, N. C. den Hollander and e. al., "Skin autofluorescence, a measure of cumulative metabolic stress and advanced glycation end products, predicts mortality in hemodialysis patients," Journal of the American Society of Nephrology, vol. 16, no. 12, p. 3687-3693, 2005.

[23] De Smet R, Dhondt A, Eloot S, Galli F, Waterloos MA, Vanholder R. Effect of the super-flux cellulose triacetate dialyser membrane on the removal of non-protein-bound and protein-bound uraemic solutes. Nephrol Dial Transplant. 2007; 22(7):2006-2012.

**Claims**

1. A device for assessing an concentration of free pentosidine in a biofluid, such as spent dialysate, comprising ultraviolet light means for generating excitation light and guiding said light into said biofluid, said light causing a flouresence emission of at least free pentosidine in said biofluid, ultraviolet sensor means for receiving and sensing said fluoresence light emission, and processing means for calculating concentration of free pentosidine based on intensity of said fluoresence light emission, **characterized in that**

said ultraviolet light means are adapted to generate excitation light with wavelength from 330 to 355nm, and said ultraviolet sensor means are adapted to sense fluoresence emission light with wavelenght is from 360 to 385 nm, preferably 363 nm.

2.  A device according to claim 1, wherein said excitation light has wavelength 345 to 355 nm, preferably 350 nm.

3.  A device according to claims 1 to 2, wherein said fluoresence emission light has wavelenght 363nm.

4.  A device according to claims 1 to 3, wherein said device is additionally adapted to generate excitation lights with second wavelength of 245 to 255 nm, preferably 250 nm and with third wavelength 425 to 435 nm, preferably 430 nm, and said ultraviolet sensor means are adapted to additionally receive second and third fluoresence emission light with wavelengths 320-322 nm, preferably 321nm and 462 to 464 nm, preferably 463nm, respectively.

5.  A device according to claim 4, wherein said device is additionally adapted to calculate said pentosidine concentration according to formula:

$$a+(b*Ex350/Em363)+(c*Ex250/Em321)+(d*Ex430/Em463),$$

where a, b, c and d are coeficients with values between -1 and 1

6.  A device according to claims 1 to 5, comprising means for receiving flowing spent dialysate.

7.  A method for assessing a concentration of a free pentosidine in a biofluid, such as spent dialysate, comprising:

    introducing ultraviolet excitation light with wavelength from 330 to 355nm, preferably 330 to 350 nm, most preferably, 350nm, into said biofluid, said light causing a fluoresence emission of at least free pentosidine in said biofluid,
    receiving said fluoresence light emission with wavelength 360 to 385nm, preferably 363nm from said biofluid, and
    determining said concentration of said free pentosidine from the intensity of said fluoresence light emission.

8.  A method according to claim 7, wherein said ultraviolet excitation light has wavelength from 330 to 350nm.

9.  A method according to claim 7, wherein said ultraviolet excitation light has wavelength 350nm.

10. A method according to claims 7 to 9, wherein said fluoresence light has wavelength 363nm.

11. A method according to claims 7 to 10, comprising introducing additionally excitation lights with second wavelength of 245 to 255 nm, preferably 250 nm and with third wavelength 425 to 435 nm, preferably 430 nm, and said ultraviolet sensor means are adapted to additionally receive second and third fluoresence emission light with wavelengths 320-322 nm, preferably 321nm and 462 to 464 nm, preferably 463nm, respectively..

12. A method according to claim 11, calculating said pentosidine concentration according to formula:

$$a+(b*Ex350/Em363)+(c*Ex250/Em321)+(d*Ex430/Em463),$$

where a, b, c and d are coeficients with values between -1 and 1

Fig 1

Fig 2

Fig 3

Fig 4

Fig 5

Fig 6

Fig. 7

Europäisches
Patentamt

European
Patent Office

Office européen
des brevets

# EUROPEAN SEARCH REPORT

Application Number

EP 18 16 0068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2013/193347 A1 (FRIDOLIN IVO [EE] ET AL) 1 August 2013 (2013-08-01) * paragraphs [0001], [0017], [0021], [0028], [0044], [0046], [0047], [0049] * <br> * claims 2,10 * | 1-12 | INV. G01N21/64 A61M1/16 |
| X | SLOWIK-ZLKA D ET AL: "A sensitive and specific HPLC method for the determination of total pentosidine concentration in plasma", JOURNAL OF BIOCHEMICAL AND BIOPHYSICAL MET, AMSTERDAM, NL, vol. 61, no. 3, 30 November 2004 (2004-11-30), pages 313-329, XP004663693, ISSN: 0165-022X, DOI: 10.1016/J.JBBM.2004.06.002 | 1,2,7-10 | |
| Y | * page 314, paragraph 4 * * page 317, point 2.6 * * page 318, paragraph 2 * * figures 6,7 * * title * | 1-12 | |
| Y | CASSIANO RANZAN ET AL: "Fluorescence Spectroscopy as a Tool for Ethanol Fermentation On-line Monitoring", PROCEEDINGS OF THE 17TH WORLD CONGRESS THE INTERNATIONAL FEDERATION OF AUTOMATIC CONTROL; SEOUL, KOREA; JULY 6-11, 2008., vol. 45, no. 15, January 2012 (2012-01), pages 940-945, XP055489409, Red Hook, NY ISSN: 1474-6670, DOI: 10.3182/20120710-4-SG-2026.00166 ISBN: 978-1-123-47890-7 * page 942, paragraph 2 * | 5,11,12 | |

TECHNICAL FIELDS SEARCHED (IPC)

G01N
A61M

The present search report has been drawn up for all claims

| Place of search | Date of completion of the search | Examiner |
|---|---|---|
| Munich | 23 July 2018 | Brauer, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                          

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 0068

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2018

| Patent document cited in search report | Publication date | Patent family member(s) | Publication date |
|---|---|---|---|
| US 2013193347 A1 | 01-08-2013 | AU 2011274009 A1 | 31-01-2013 |
| | | BR 112012033625 A2 | 25-10-2016 |
| | | CA 2837488 A1 | 05-01-2012 |
| | | CN 102985822 A | 20-03-2013 |
| | | EP 2585830 A1 | 01-05-2013 |
| | | JP 5957450 B2 | 27-07-2016 |
| | | JP 2013534630 A | 05-09-2013 |
| | | KR 20130038347 A | 17-04-2013 |
| | | NZ 605685 A | 31-10-2014 |
| | | US 2013193347 A1 | 01-08-2013 |
| | | WO 2012000521 A1 | 05-01-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2585830 A

### Non-patent literature cited in the description

- **D. R. SELL ; V. M. MONNIER.** *Structure Elucidation of a Senescence Cross-Link from Human Extracellular Matrix,* 1989, vol. 36, 21597-21602
- **VANHOLDER.** *Uremic Toxin - Data Base,* September 2009, http://www.uremic-toxins.org/DataBase.html.
- **M. E. SULIMAN ; O. HEIMBURGER ; P. BARANY ; B. ANDERSTAM ; R. PECOITS-FILHO ; E. R. AYALA ; A. R. QURESHI ; I. FEHRMAN-EKHOLM, L. B ; P. STENVINKEL.** *Plasma Pentosidine Is Associated with Inflammation and Malnutrition in End-Stage Renal Disease Patients Starting on Dialysis Therapy,* 2003, vol. 6, 1614-1622
- **T. MIYATA ; Y. UEDA ; A. YOSHIDA ; S. SUGIYAMA ; Y. IIDA ; M. JADOUL ; K. MAEDA ; K. KUROKAWA ; C. VAN YPERSELE DE STRIHOU.** Clearance of pentosidine, an advanced glycation end product, by different modalities of renal replacement therapy. *Kidney International,* 1997, vol. 51 (3), 880-887
- **T. MIYATA ; Y. UEDA ; T. SHINZATO ; Y. IIDA ; S. TANAKA ; K. KUROKAWA ; C. VAN YPERSELE DE STRIHOU ; K. MAEDA.** Accumulation of albumin linked and free-form pentosidine in the circulation of uremic patients with end-stage renal failure: Renal implications in the pathophysiology of pentosidine. *Journal of the American Society of Nephrology,* 1996, vol. 7, 1198-1206
- **M. A. FRIEDLANDER ; Y. C. WU ; A. ELGAWISH ; V. M. MONNIER.** Early and advanced glycosylation end products. Kinetics of formation and clearance in peritoneal dialysis. *Journal of Clinical Investigation,* 1996, vol. 97, 728-735
- **G. STEIN ; S. FRANKE ; A. MAHIOUT ; S. SCHNEIDER ; H. SPERSCHNEIDER ; S. BORST ; J. VIENKEN.** Influence of dialysis modalities on serum AGE levels in end-stage renal disease patients. *Nephrology Dialysis Transplantation,* 2001, vol. 16 (5), 999-1008
- **M. ADLER.** Pentosidine Analysis Method Implementation in Chromatography Laboratory of TUT Department of Health Technologies. *TTU,* 2017
- **A. MACHOWSKA ; J. SUN ; A. R. QURESHI ; N. ISOYAMA ; P. LEURS ; B. ANDERSTAM ; O. HEIMBURGER ; P. BARANY ; P. STENVINKEL ; B. LINDHOLM.** Plasma Pentosidine and Its Association with Mortality in Patients with Chronic Kidney Disease. *PLOS one,* 2016
- **M. E. SULIMAN ; O. HEIMBÜRGER ; P. BARANY ; B. ANDERSTAM ; R. PECOITS-FILHO ; A. E. RODRIGUEZ.** Plasma pentosidine is associated with inflammation and malnutrition in end-stage renal disease patients starting on dialysis therapy. *Journal of the American Society of Nephrology,* 2003, vol. 6 (16), 1614-1622
- **A. HEIDLAND ; K. SEBEKOVA ; R. SCHINZEL.** Advanced glycation end products and the progressive course of renal disease. *American Journal of Kidney Diseases,* 2001, S100-S106
- **J. NI ; X. YUAN ; J. GU ; X. YUE ; X. GU ; R. H. NAGARAJ ; J. W. CRABB.** Plasma Protein Pentosidine and Carboxymethyllysine, Biomarkers for Age-related Macular Degeneration. *Molecular and Cellular Proteomics,* 2009, vol. 8 (8), 1921-1933
- **F. UHLIN ; J. HOLMAR ; P. YNGMAN-UHLIN ; A. FERNSTRÖM ; I. FRIDOLIN.** Optical Estimation of Beta 2 Microglobulin During Hemodiafiltration - Does It Work?. *Blood Purification,* 2015, vol. 2 (40), 113-119
- **I. FRIDOLIN ; M. MAGNUSSON ; L. G. LINDBERG.** On-Line Monitoring of Solutes in Dialysate Using Absorption of Ultraviolet Radiation: Technique Description. *The International journal of Artificial Organs,* 2002, vol. 8 (25), 748-761
- **S. KALLE ; R. TANNER ; J. ARUND ; R. TOMSON ; M. LUMAN ; I. FRIDOLIN.** 4-Pyridoxic Acid in the Spent Dialysate: Contribution to Fluorescence and Optical Monitoring. *PLOS one,* 2016
- **K. H. ESBENSEN.** Multivariate data analysis - in practice, Oslo, Norway. *CAMO Process AS,* 2009
- Master thesis: Software for regression analysis of biofluids. **J. MICHELIS.** spectrophotometric data. Tallinn University of Technology, 2016, 59

- **J. M. BLAND ; D. G. ALTMAN.** Statistical methods for as - sessing agreement between two methods of clinical measurement. *Lancet,* 1986
- **S. KALLE ; R. TANNER ; I. FRIDOLIN ; M. LUMAN.** Association between skin autofluorescence and fluorescence of spent dialysate. *Nephrology Dialysis Transplantation, 54th ERA-EDTA Congress,* 03 June 2017
- **M. E. SULIMAN ; O. HEIMBÜRGER ; P. BÁRÁNY ; B. ANDERSTAM ; R. PECOITS-FILHO ; E. RODRIGUEZ AYALA ; A. R. QURESHI ; I. FEHRMAN-EKHOLM ; B. LINDHOLM ; P. STEN-VINKEL.** Plasma pentosidine is associated with inflammation and malnutrition in end-stage renal disease patients starting on dialysis therapy. *Journal of the American Society of Nephrology,* 2003, vol. 6 (14), 1614-1622
- **D. SLOWIK-Z ẎLKA ; K. SAFRANOW ; V. DZIEDZIEJKO ; H. BUKOWSKA ; K. CIECHANOWSKI ; D. CHLUBEK.** A sensitive and specific HPLC method for the determination of total pentosidine concentration in plasma. *Journal of Biochemical and Biophysical Methods,* 2004, vol. 61, 313-329
- **R. MEERWALDT ; J. W. HARTOG ; R. GRAAFF ; R. J. HUISMAN ; T. P. LINKS ; N. C. DEN HOLLANDER.** Skin autofluorescence, a measure of cumulative metabolic stress and advanced glycation end products, predicts mortality in hemodialysis patients. *Journal of the American Society of Nephrology,* 2005, vol. 16 (12), 3687-3693
- **DE SMET R ; DHONDT A ; ELOOT S ; GALLI F ; WATERLOOS MA ; VANHOLDER R.** Effect of the super-flux cellulose triacetate dialyser membrane on the removal of non-protein-bound and protein-bound uraemic solutes. *Nephrol Dial Transplant,* 2007, vol. 22 (7), 2006-2012